# EUROPEAN PATENT APPLICATION

(11) **EP 1 375 675 A2**
(43) Date of publication of application: **02.01.2004**
(21) Application number: 03014164.2
(22) Date of filing: 24.06.2003
(51) Int. Cl.: C12Q 1/68, C03C 17/30, C03C 17/34, C07B 61/00, G01N 33/543, G01N 33/552

(54) **Substrate for immobilization of a biological substance or chemical substance, method of immobilizing a biological substance or chemical substance, and a method of coating such a substrate**

(30) Priority: 28.06.2002 JP 2002189554
(71) Applicant: DNA Chip Research Inc., Yokohama-shi, 230-0045 Kanagawa (JP); Hitachi Software Engineering Co., Ltd., Yokohama-shi, Kanagawa 230-0045 (JP)
(72) Inventor: Komatsu, Yasuo, c/o DNA Chip Research Inc., Yokohama-shi, Kanagawa 230-0045 (JP); Matsubara, Kenichi, c/o DNA Chip Research Inc., Yokohama-shi, Kanagawa 230-0045 (JP); Ohtsuka, Eiko, c/o DNA Chip Research Inc., Yokohama-shi, Kanagawa 230-0045 (JP); Nonaka, Ken, c/o DNA Chip Research Inc., Yokohama-shi, Kanagawa 230-0045 (JP); Yurino, Noriko, Hitachi Software Engineering CoLtd, Tokyo 140-0002 (JP)
(74) Representative: Liesegang, Roland, Dr.-Ing.

(57) **Abstract**

A coated substrate for immobilizing a biological substance etc. at high density with low background value. The substrate has a cross-linking reagent represented by general formula A-L-B (wherein, A and B represent an identical or different group which reacts with an active group of the coupling agent comprising an active group, selected from an active ester group, isothiocyanate group, isocyanate group, imidazole group, carbodiimide group or aldehyde group, and wherein L is a linking group linking A and B, selected from a straight chain alkyl group, aryl group, allyl group or alkyl group having an amide group) bound thereonto by means of a coupling agent comprising an active group. An biological substance or the like is immobilized on the substrate.

## Description

### TECHNICAL FIELD

The present invention relates to a substrate adapted for immobilization of a biological substance on a fixed substrate including glass, plastic, ceramic or the like, a method of immobilizing a biological substance on such substrates, and a method of coating a substrate.

### BACKGROUND ART

DNA chips include cDNA chips where long chain PCR products are immobilized on a substrate and oligonucleotide chips where single chain oligonucleotides having chain lengths of 100 bases or less are immobilized. Because cDNA chips are derived from PCR products, it has double-stranded DNA immobilized thereon, and thus incorrect hybridization (cross-hybridization) of a sample derived from an analyte can occur.

Further, oligonucleotide chips in current use can be broadly divided into those involving a method of synthesizing oligonucleotides on a glass substrate, and those involving a method of immobilizing a synthesized oligonucleotide onto a glass substrate. Oligonucleotide synthesis onto a chip has the advantage that it enables synthesis of many types of oligonucleotide. However, because post-synthesis purification is omitted, there is the disadvantage that purity is low. Further, with this method of synthesis on glass, only single chain oligonucleotides can be synthesized. Thus for future preparation of chips having biological substances such as double-stranded DNA, saccharides, proteins or the like immobilized thereon, methods of synthesizing onto a substrate are not suitable. Thus, it is considered that high versatility will be demanded of method of spotting a synthesized oligonucleotides.

Several methods of immobilization have been reported which involve coating a substrate, of which glass is a representative example, with a special coating and spotting oligonucleotides thereon. However, these methods all present the problem of high background values which originate in the fact that luminescent pigments used to label samples adhere to a surface of the substrate in a non-specific manner. Further, there are reports that if the viscosity of a solution in which nucleic acid is dissolved is too low, a spotted droplet will spread excessively. Consequently, developments of solutions for spotting is also an important problem where spotting at high density.

Given the above background, the object of the present invention is to provide a coated substrate for immobilizing a biological substance or the like at high density with low background value, a method of immobilizing a biological substance or the like by spotting, and a method of coating a substrate.

### DISCLOSURE OF THE INVENTION

The present inventors, in order to solve the above problem, found that it was possible to prepare a substrate for immobilization of a biological substance at high concentration and with low background values, by coating a substrate using a specific linking group.

Firstly, the substrate of the present invention is a substrate having a cross-linking reagent represented by the following general formula (1) bound thereonto by means of a coupling agent comprising an active group:

A-L-B (1)

(wherein, A and B represent an identical or different group which reacts with an active group of the coupling agent comprising an active group, selected from an active ester group, isothiocyanate group, isocyanate group, imidazole group, carbodiimide group or aldehyde group, and wherein L is a linking group linking A and B, selected from a straight chain alkyl group, aryl group, allyl group or alkyl group having an amide group).

A preferred specific example of the coupling agent comprising an active group is a silane compound comprising an amino group, represented by the following formula (2): 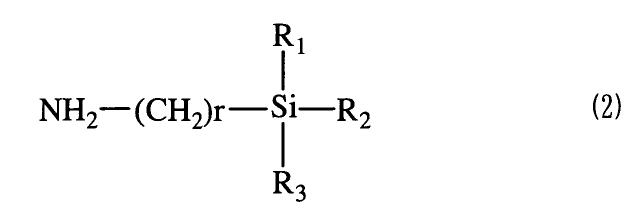 (wherein, R1, R2 and R3 represent an identical or different group being -O(CH₂)ₐCH₃ or Cl, a represents an identical or different integer from 0 to 10, and r represents an integer from 0 to 10).

A further preferred example of the coupling agent comprising an active group is a silane compound comprising an active group, represented by the following formula (3): 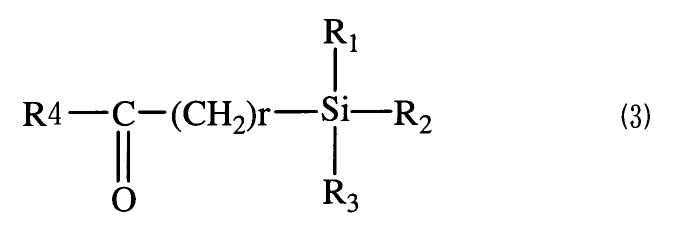 (wherein R4 represents any one of the following structures: 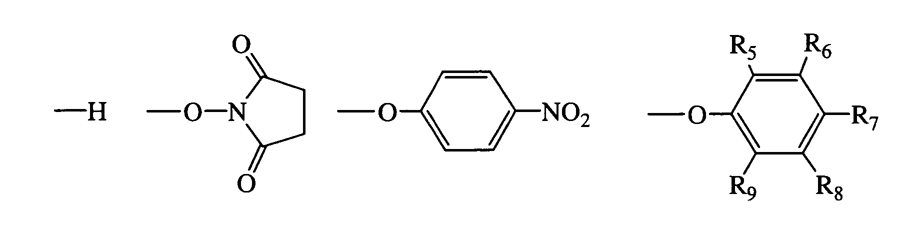 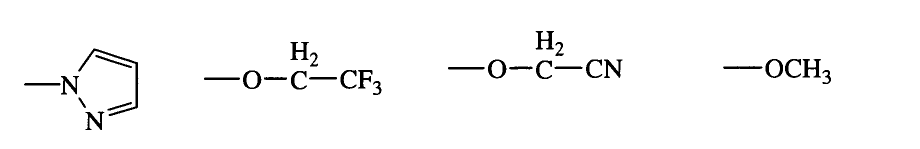 wherein, R5, R6, R7, R8 and R9 represent an identical or different atom being H, Cl or F).

A further preferred example of the coupling agent comprising an active group, is a silane compound comprising an active group, represented by the following general formula (4): 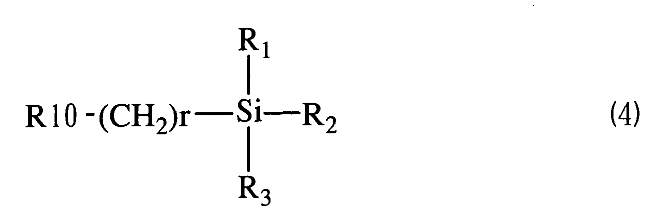 wherein R10 represents any one of the following structures:

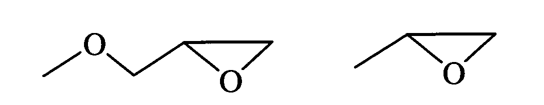

A preferred example of the cross-linking agent A-L-B of general formula (1) is any one of the following compounds: 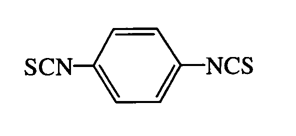 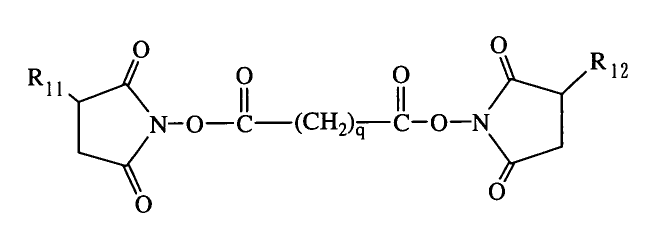 (wherein R11 and R12, represent an identical or different group being -H or -SO₃)

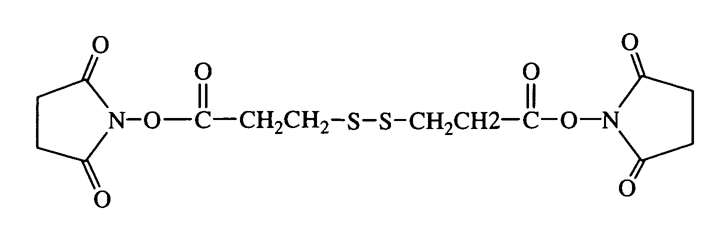

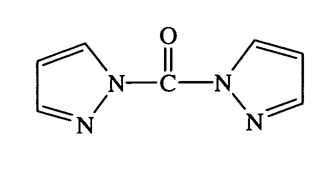

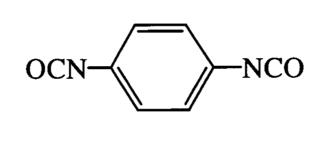

OHC(CH₂)₃CHO

Secondly, the substrate of the present invention is a substrate having a branching agent intramolecularly possessing 2 or more primary amino groups, bound thereto by means of the cross-linking reagent represented by A-L-B of general formula (1) of the substrate according to any one of the above. A broad range for number of primary amino groups within a molecule, from 2 to 64, can be used.

A preferred example of the branching agent is a compound having 2 or 3 primary amino groups, which is represented by the following general formula (5): 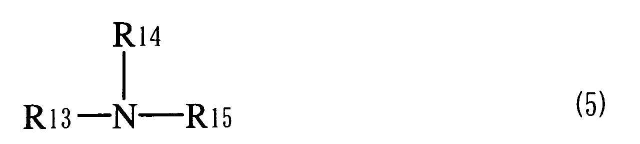 (wherein, R13, R14 and R15 represent an identical or different group selected from alkyl group having a primary amino group, alkyl group or hydrogen atom, provided that 2 or 3 are alkyl groups having a primary amino group).

Here, it is preferable that R13, R14 and R15 of the general formula (5) are alkyl amino groups represented by the following general formula (6):

NH₂(CH₂)_{b}(NH)_{c}(CH₂)_{d}(NH)ₑ(CH₂)_{f}- (6)

(wherein, b, d and f represent identical or different integers from 0 to 10, c and e represent identical or different integers from 0 to 1, provided that cases where b, d and f are all 0 is not included).

Particularly preferred examples of the branching agent include Tris(3-aminopropyl)amine and Tris(2-aminoethyl)amine. A preferred example is polypropylenimine tetraamine dendrimer having 4 primary amino groups represented by the following formula:

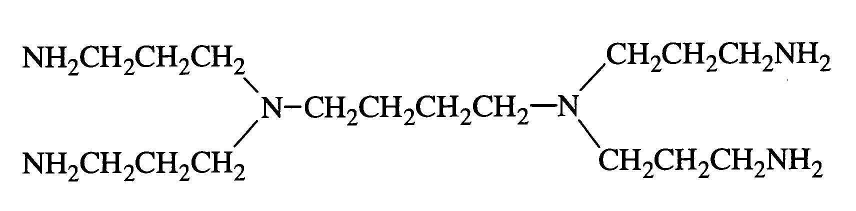

A further preferred example of the branching agent is a PAMAM compound intramolecularly possessing numerous primary amino groups. Here, examples of a PAMAM compound include Starburst Generation 1 (a product name of Aldrich Chemical Co.) which has 8 primary amino groups in a molecule thereof, and Starburst Generation 2 (a product name of Aldrich Chemical Co.) which has 16 primary amino groups in a molecule thereof.

Further, preferred examples of the branching agent include polyamide dendrimer compounds having 2 or more primary amino groups. Polyamide dendrimer compounds are compounds having an amide group within a branch chain, and a primary amino group at their terminus. There are compounds having 4, 8, 16, 32 and 64 primary amino groups, and are respectively named Generation 0.0, 1.0, 2.0, 3.0, 4.0 (product names of Aldrich Chemical Co.). A preferred example of a poly amide dendrimer having 4 primary amino groups is the following:

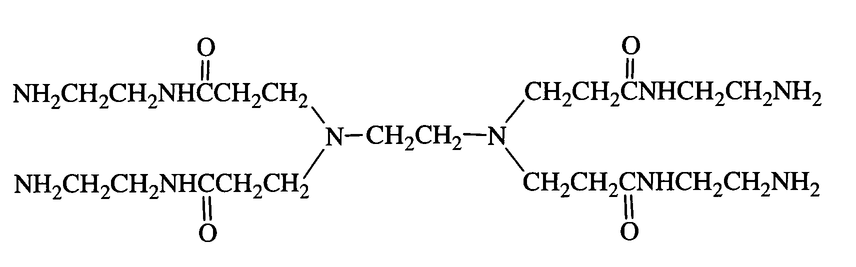

Thirdly, the substrate according to the present invention is a substrate according to the second invention described above, having a cross-linking reagent of the following general formula (1) further bound thereonto by means of the branching agent intramolecularly possessing 2 or more primary amino groups:

A-L-B (1)

(wherein, A and B represent an identical or different group which reacts with an active group of the coupling agent comprising an active group, selected from an active ester group, isothiocyanate group, isocyanate group, imidazole group, carbodiimide group or aldehyde group, and wherein L is a linking group linking A and B, selected from a straight chain alkyl group, aryl group, allyl group or alkyl group having an amide group).

Fourthly, the present invention is a substrate for immobilization of a biological substance or chemical substance, which has a biological substance selected from nucleic acid, protein, saccharide, glycoprotein, blood, body fluid or the like, or a chemical substance selected from a medicament, environmental hormone, PCB or the like, being an object of binding, immobilized thereon by means of the cross-linking agent of the any one of the above substrates, represented by general formula (1) A-L-B.

Fifthly, the present invention is a method of immobilizing a biological substance or chemical substance which comprises immobilizing a biological substance selected from nucleic acid, protein, saccharide, glycoprotein, blood, body fluid or the like, or a chemical substance selected from a medicament, environmental hormone, PCB or the like, onto any of the substrates described above.

Sixthly, the present invention is a method of coating a substrate which comprises reacting a coupling agent comprising an active group on a substrate, and then reacting a cross-linking reagent represented by the following general formula (1):

A-L-B (1)

(wherein, A and B represent an identical or different group which reacts with an active group of the coupling agent comprising an active group, selected from an active ester group, isothiocyanate group, isocyanate group, imidazole group, carbodiimide group or aldehyde group, and wherein L is a linking group linking A and B, selected from a straight chain alkyl group, aryl group, allyl group or alkyl group having an amide group).

A preferred example of the coupling agent comprising an active group is a silane compound comprising an amino group, represented by the following general formula (2): 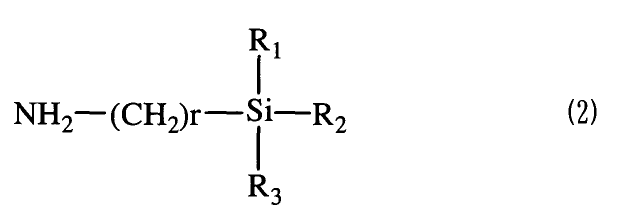 (wherein, R1, R2 and R3 represent an identical or different group being -O(CH₂)ₐCH₃ or Cl, a represents an identical or different integer from 0 to 10, and r represents an integer from 0 to 10).

A further preferred example of the coupling agent comprising an active group is a silane compound comprising an amino group, represented by the following general formula (3): 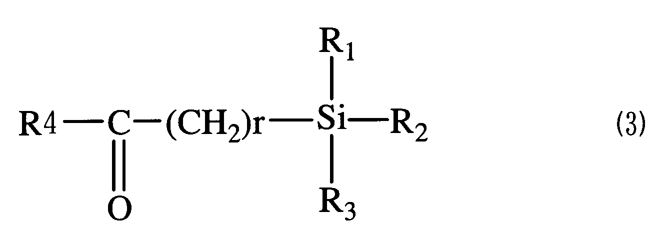 (wherein R4 represents any one of the following structures: 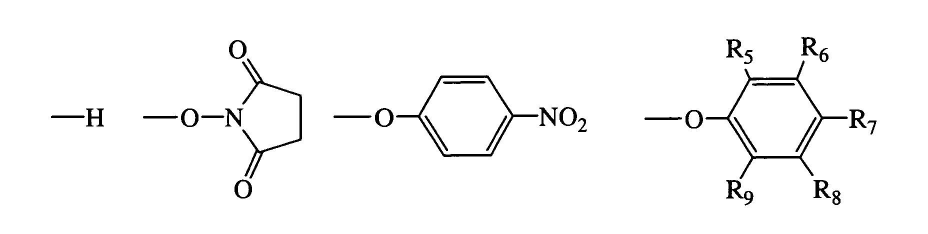 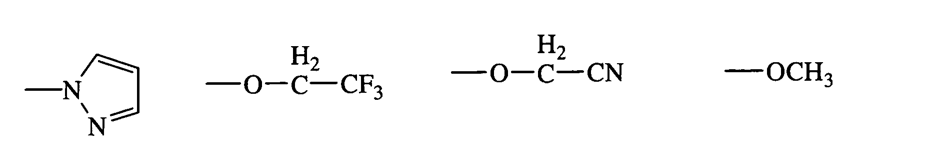 wherein, R5, R6, R7, R8 and R9 represent an identical or different atom being H, Cl or F).

A further preferred example of the coupling agent comprising an active group, is a silane compound comprising an active group, represented by the following general formula (4): 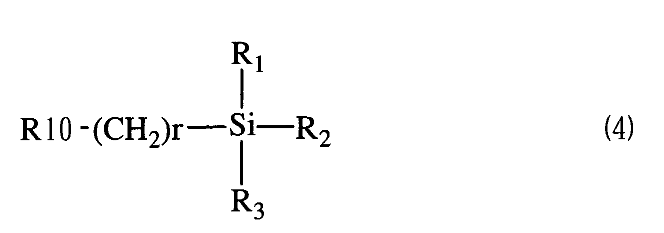 wherein R10 represents any one of the following structures:

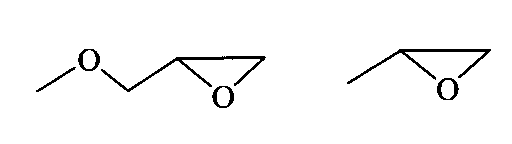

A preferred example of the cross-linking reagent A-L-B of general formula (1) is any one of the following compounds: 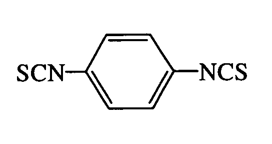 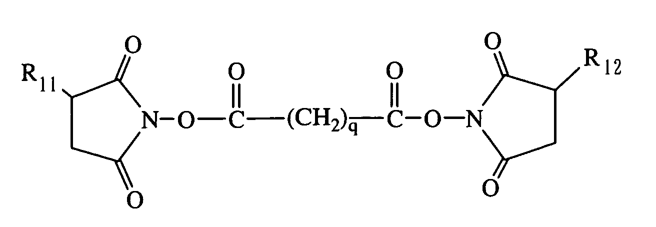 (wherein R11 and R12, represent an identical or different group being -H or -SO₃)

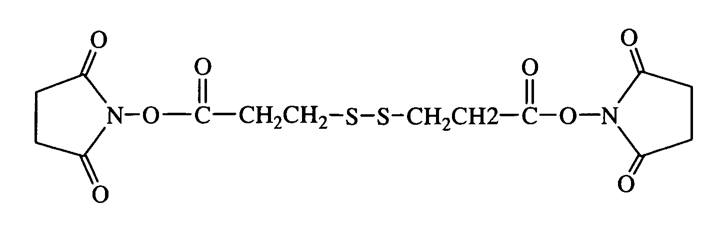

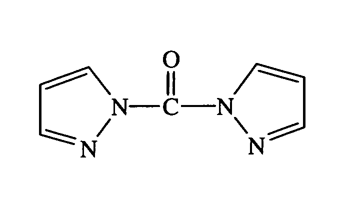

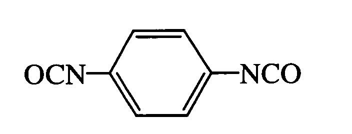

OHC(CH₂)₃CHO

Seventhly, the present invention is a method of coating a substrate which comprises reacting a branching agent intramolecularly possessing 2 or more primary amino groups with the substrate manufactured by the sixth invention described above.

A preferred example of the branching agent is a compound having 2 or 3 primary amino groups represented by the following general formula (5): 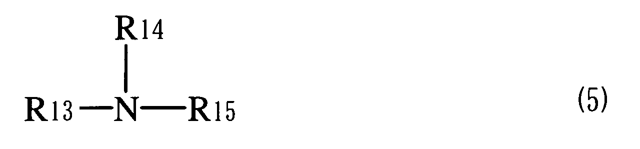 (wherein, R13, R14 and R15 are an identical or different group selected from alkyl group having a primary amino group, alkyl group or hydrogen atom, provided that 2 or 3 are alkyl groups having a primary amino group).

Here, it is preferable that R13, R14 and R15 of the general formula (5) are alkyl amino groups represented by the following general formula (6):

NH₂(CH₂)_{b}(NH)_{c}(CH₂)_{d}(NH)ₑ(CH₂)_{f}- (6)

(wherein, b, d and f represent identical or different integers from 0 to 10, c and e represent identical or different integers from 0 to 1, provided that cases where b, d and f are all 0 is not included).

Particularly preferred examples of the branching agent include Tris(3-aminopropyl)amine and Tris(2-aminoethyl)amine. A preferred example is polypropylenimine tetraamine dendrimer having 4 primary amino groups represented by the following formula:

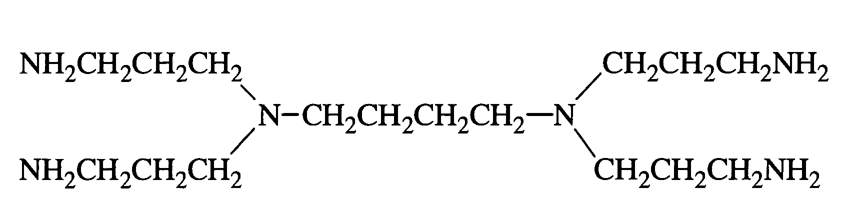

A further preferred example of the branching agent is a PAMAM compound intramolecularly possessing numerous primary amino groups. Here, examples of a PAMAM compound include Starburst Generation 1 (a product name of Aldrich Chemical Co.) which has 8 primary amino groups in a molecule thereof, and Starburst Generation 2 (a product name of Aldrich Chemical Co.) which has 16 primary amino groups in a molecule thereof.

Further, preferred examples of the branching agent include polyamide dendrimer compounds having 2 or more primary amino groups. Polyamide dendrimer compounds are compounds having an amide group within a branch chain, and a primary amino group at their terminus. There are compounds having 4, 8, 16, 32 and 64 primary amino groups, and are respectively named Generation 0.0, 1.0, 2.0, 3.0, 4.0 (product names of Aldrich Chemical Co.). A preferred example of a poly amide dendrimer having 4 primary amino groups is the following:

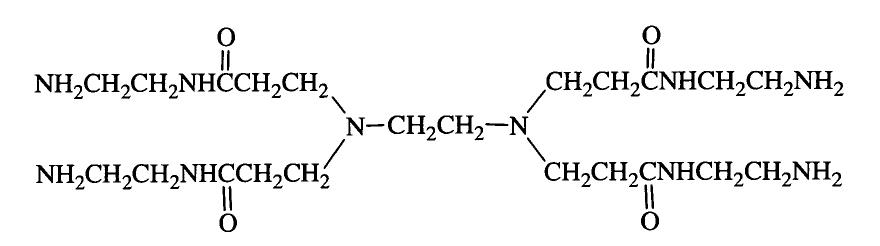

Eighthly, the present invention is a method of coating a substrate which comprises further reacting a cross-linking reagent of the following general formula (1) with the substrate according to the seventh invention described above:

A-L-B (1)

(wherein, A and B represent an identical or different group which reacts with an active group of the coupling agent comprising an active group, selected from an active ester group, isothiocyanate group, isocyanate group, imidazole group, carbodiimide group or aldehyde group, and wherein L is a linking group linking A and B, selected from a straight chain alkyl group, aryl group, allyl group or alkyl group having an amide group).

As a substrate of the present invention, a substrate conventionally known as a substrate for immobilizing a biological substance or medicament can be used, such as a plate of glass, plastic or the like, or beads manufactured from glass, plastic or the like.

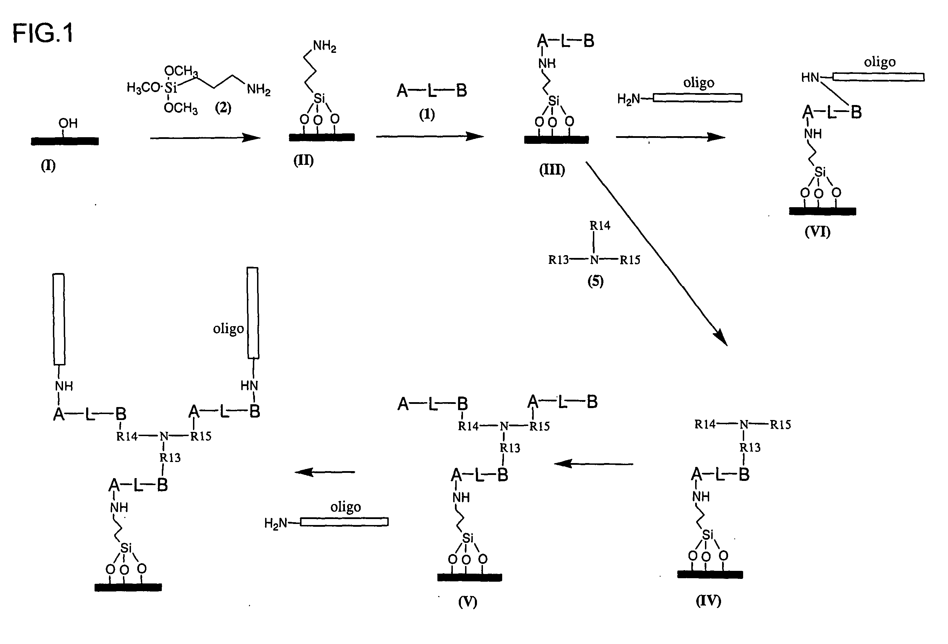

Figures 1 to 3, represent the flow of the present invention as chemical formulae.

In Fig. 1. a silane compound comprising an amino group, as coupling agent comprising an active group, is reacted (II) with a hydroxyl group present on a surface of a substrate (I). Next, this is reacted (III) with a cross-linking reagent having a linking group L represented by general formula (1) A-L-B. Oligonucleic acid is immobilized (VI) on the obtained substrate.

Further, a compound having 2 or 3 primary amino groups represented by the following general formula (5), which is a branching agent, is reacted (IV) with substrate (III).

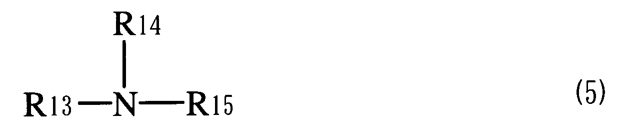

The cross-linking reagent according to general formula (1) is further reacted (V) with this branching agent, and 2 oligonucleic acids are immobilized in respect of 1 cross-linking reagent.

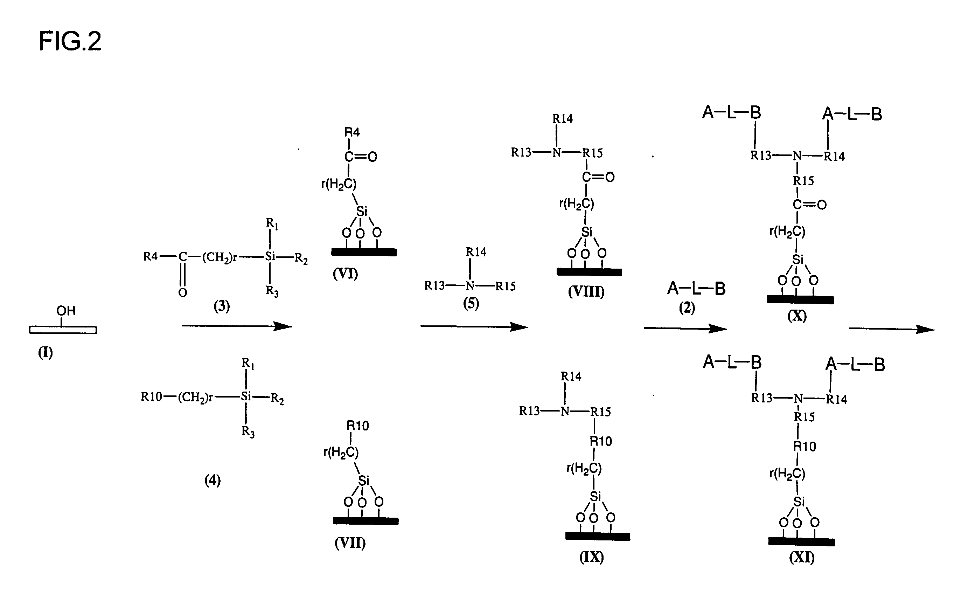

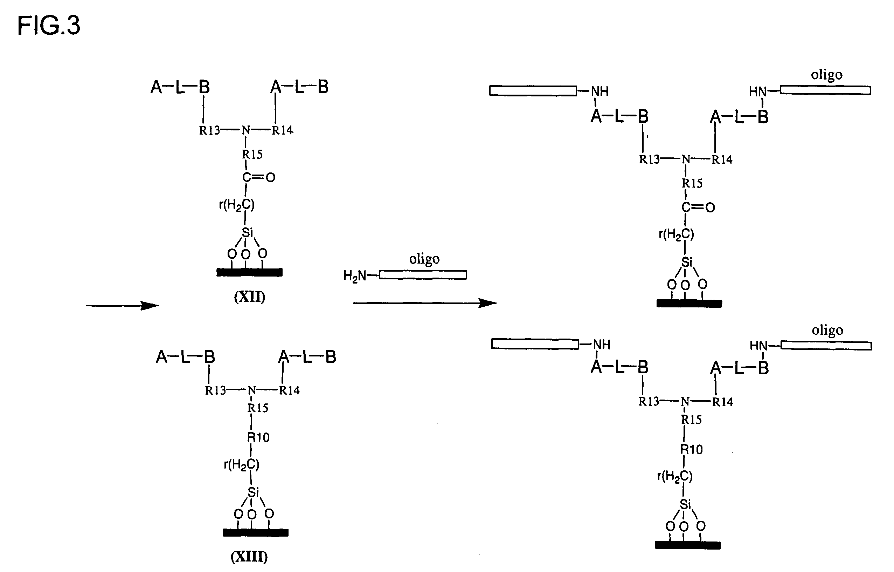

In Figures 2 and 3, the following coupling agent comprising an active group are reacted (VI)(VII) with a hydroxyl group present on a surface of substrate (I): 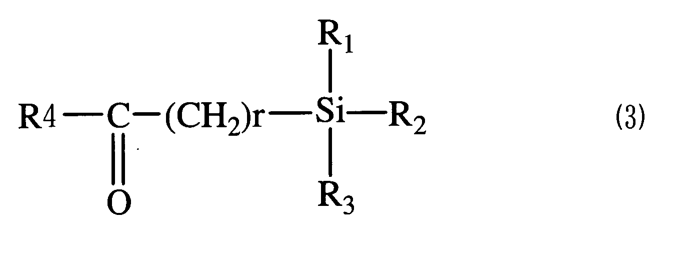 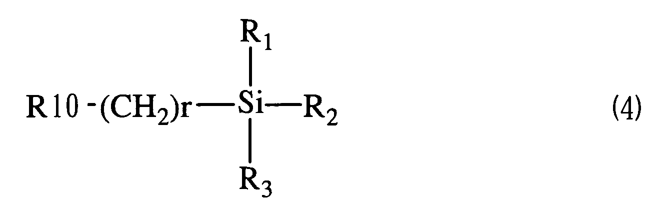 and a compound represented by the following general formula (5) which is a branching agent intramolecularly possessing 2 or 3 primary amino groups is reacted therewith (VIII)(IX):

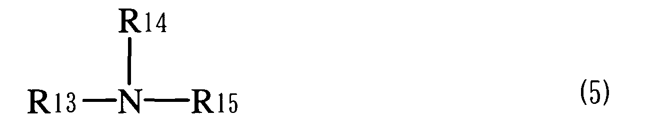

Further a cross-linking reagent according to general formula (1) is reacted with this branching agent (V)(XI) and 2 oligonucleic acids are immobilized in respect of 1 cross-linking reagent respectively.

### BRIEF DESCRIPTION OF THE DRAWINGS

Fig. 1 shows chemical formulae indicating the flow of the present invention.
Fig. 2 shows chemical formulae indicating the flow of the present invention.
Fig. 3 shows chemical formulae indicating the flow of the present invention.
Fig. 4 is a schematic representation of the Examples of the present invention

### BEST MODE FOR CARRYING OUT THE INVENTION

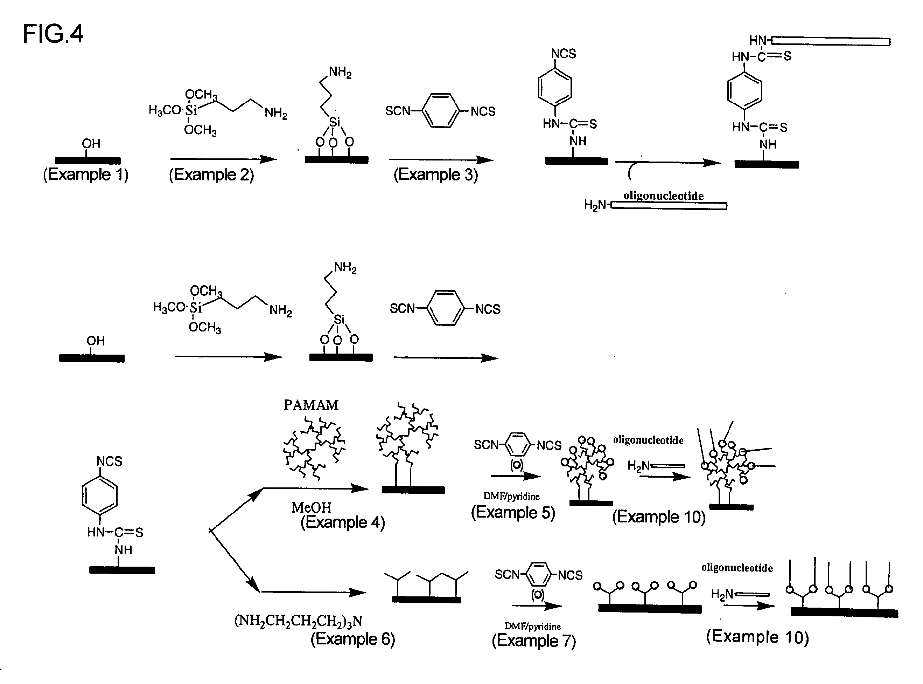

Below, the present invention is explained by way of Examples. Figure 4 is a schematic overview of these Examples.

### Example 1: Washing of slide glasses

Slide glasses (10 to 20 slides) were immersed for 15 minutes in 10% sodium hydroxide-water solution (200mL), and then washed with water (200mL, twice), 1% HCl-water solution (200mL), and water (200mL, twice) in that order. The slide glasses were then immersed in methanol (200mL), subjected to ultrasonic wash for 5 minutes, dried by centrifugation, and further dried at 180°C for 3 hours.

### Example 2: Aminopropyl silanation

The dried slide glasses were immersed in 3-aminotrimethoxy silane (13mL), water (8mL), methanol (380mL), and stirred for at least 5 hours at room temperature. Thereafter, the slide glasses were removed, washed twice with methanol (200mL), and after centrifugation dried for 3 hours at 180°C.

### Example 3: Isothiocyanation

1,4-phenylene diisothiocyanate (1836mg) was first dissolved in 10% pyridine/dimethyl formamide solution (190mL), the slide glasses were placed therein, and stirred for 16 hours at room temperature. The slide glasses were taken out, washed in dimethyl formamide (200mL), dichloro methane (200mL) and methanol (200mL) in that order, dried under reduced pressure at room temperature and made available for isothiocyanation in Example 5.

### Example 4: Dendrimerization

The following reaction was performed using PAMAM (Aldrich) as a dendrimer: Slide glasses aminated in Example 3 were placed in a glass vessel and 10% PAMAM methanol solution (130µL) was dripped thereonto. The vessel was sealed and reaction allowed to proceed for 5 hours at 37°C. Thereafter, the slide glasses were washed twice with methanol (200mL), washed once with acetone (200mL), and dried under reduced pressure at room temperature for 1 hour.

### Example 5: Isothiocyanation after dendrimer coating

The slide glasses obtained in Example 4 were isothiocyanated by performing the same reaction as in Example 3, and made available for oligonucleotide spotting.

### Example 6: Tris (3-aminopropyl)amination

The slide glass obtained in Example 3, was laid out in a glass vessel, and 20 or 30% Tris (3-aminopropyl) amine/methanol solution (130µL) was dripped thereonto. The vessel was sealed, and reaction allowed to continue for 5 hours at 37°C. Thereafter, the slide glasses were washed twice with methanol (200mL), once with acetone (200mL) and then dried under reduced pressure at room temperature for 1 hour.

### Example 7: Isothiocyanation after 3-aminopropyl amine coating

The slide glasses obtained in Example 6 were isothiocyanated by performing a reaction similar to that of Example 3, and these were used for spotting of oligonucleotides.

### Example 8: polypropylenimine tetraamine dendrimerization

The slide glass obtained in Example 3, was laid out in a glass vessel, and 20 or 30% polpropylenimine tetraamine dendrimer/methanol solution (130µL) was dripped thereonto. The vessel was sealed, and reaction allowed to continue for 5 hours at 37°C. Thereafter, the slide glasses were washed twice with methanol (200mL), once with acetone (200mL) and then dried under reduced pressure at room temperature for 1 hour.

### Example 9: Isothiocyanation after polypropylenimine tetraamine dendrimer coating

The slide glasses obtained in Example 8 were isothiocyanated by performing a reaction similar to that of Example 3, and these were used for spotting of oligonucleotides.

### Example 10: Spotting of oligonucleotides onto slide glass and binding reaction

An oligonucleotide library (80pmol; manufactured by MWG) was dissolved in sterilized water (4µL), and mixed with spot solution (4µL; 1M carbonate buffer (pH9.0): saturated Wako gel C-300 (product name of Wako Pure Chemical Industries, Ltd)-water solution =1:1) and spotted onto the coated slide glass obtained in Examples 5, 7 and 9 by a spotter (SPBI02000, a product name of Hitachi Software Engineering; SPBIO is a registered trademark). After spotting, a filter paper was laid out in a tight box and wetted with 300mM disodium hydrogen phosphate-water solution. Spotted slide glass were then placed in the tight box in a manner such that the solution did not adhere thereto and then the tight box was sealed. After allowing to stand at 37°C for 16 hours, slide glasses were removed from tight box for blocking the surface of the slide glass..

### Example 11: Blocking of oligonucleotide spot glasses

Slide glasses were washed with 0.1 % Triton X (200mL) at room temperature for 5 minutes, with 0.05% HCl aqueous solution (200mL) at room temperature for 2 minutes, with 0.1M KCl (200mL) at room temperature for 10 minutes, and with sterilized water (200mL) at room temperature for 1 minute wash.

Next, blocking was performed by immersing the glasses in a 1M Trisglycine solution (pH=9.0) or 1M ethanolamine aquaous solution and stirring for 1 hour at room temperature. The glasses were then washed twice with sterilized water, dried in a draft, and refrigerated.

### (Reference Example) Reverse transcription reaction

### Reverse transcription reaction using total RNA derived from mouse liver

Total RNA (100µg) derived from mouse liver was dissolved in oligo dt primer (2µl;0.5µg/µL) for reverse transcription reaction and sterilized water to a total volume of 30µl, and this was heated at 70°C for 5 minutes, and 42°C for 2 minutes. Thereafter, a pre-prepared solution comprising a pigment (50µL; reverse transcription reaction buffer (×5, 250mM Tris-HCl pH8.3, 375 mM KCl, 15 mM MgCl₂, 50 mM DTT: 16µL), 0.1M dithiothreitol (8µL), 10× dNTP solution (8µL; 2mM dTTP, 5mM dATP, 5mM dCTP, 5mM dGTP), 1mM Cy3-dUTP or Cy5-dUTP (8µL), RNase inhibitor (10µL; 40units/µL)) was mixed with the RNA solution. Next, a reverse transcription enzyme (1µL, 200units; Superscript II RNase H;GIBCOBRL) was added, and the solution was heated at 42°C. After 1 hour, a further 0.5µL of the above reverse transcription enzyme was added, and reaction was allowed to proceed for 1 hour at 42°C. To this reaction solution, sterilized water (40µL), 0.5M EDTA (10µL) and 1N NaOH (20µL) were added, and heating performed at 65°C for 30 minutes. 1N HCl (15µL) was added to neutralize, and the solution was cooled with ice. After performing ethanol precipitation, sterilized water (50µL) was added, and desalting was performed with Microcon P30 (product name of BIO RAD). The elute was concentrated with Speed Vac.

### Example 12: Hybridization with cDNA prepared from mouse liver total RNA

20 × SSC (10µL), 10% SDS (0.4µL), and sterilized water was added to the fluorescence labeled cDNA prepared in accordance with the Reference Example to prepare a total amount of 40µL of probe solution. After gently placing this probe DNA solution on the DNA chip, a cover glass was placed on the solution, this was placed on a Kim towel dampened with 4 × SSC solution laid out in a tight box and allowed to stand for 16 hours at 40°C.

After hybridization, the chips were washed for 10 minutes with 1 × SSC-0.05% SDS solution (200mL, 40°C), for 2 minutes with 0.2 × SSC-0.1% SDS solution (200mL, 40°C), and then washed respectively with 0.2 × SSC (200mL) and 0.05 × SSC (200mL) at room temperature. After drying, detection was performed with a chip scanner (Scan Array, a product name of A Packard Bioscience Company). Cy3-dUTP or Cy5-dUTP-labeled cDNA was prepared from mouse liver-derived RNA, and result of analysis by oligonucleotide chip were results for detected wavelength: green for Cy3 and red for Cy5.

### Example 13: Confirmation of immobilization of oligonucleotides onto slide glass

Sterilized water was added 1mM Cy5-dUTP (2µL), dimethylsulfoxide (4µL), 25 mM cobalt chloride solution (4µL), reaction buffer (× 5, 1M potassium cacodylate, 25 mM Tris-HCl, 1.25mg/BSA, pH6.6; 8µL) and terminal transferase (50 units) to prepare a reaction solution having a total volume of 40µL. The entire amount was dripped onto a slide glass immediately thereafter. A cover glass was placed over the reaction solution, which was allowed to stand at 37°C. After 15 minutes, this was washed with 4 × SSC buffer (0.6M NaCl, 0.12M citric acid dihydrate), 0.1% SDS solution, and 50% ethanol-water solution, dried, and measurement performed with a detector (ScanArray).

### INDUSTRIAL APPLICABILITY

By coating a substrate in the manner of the present invention, a biological substance or the like can be immobilized at high density and with low background values

## Claims

1. A substrate having a cross-linking reagent represented by the following general formula (1) bound thereonto by means of a coupling agent comprising an active group:
A-L-B (1)
(wherein, A and B represent an identical or different group which reacts with an active group of the coupling agent comprising an active group, selected from an active ester group, isothiocyanate group, isocyanate group, imidazole group, carbodiimide group or aldehyde group, and wherein L is a linking group linking A and B, selected from a straight chain alkyl group, aryl group, allyl group or alkyl group having an amide group).

2. A substrate according to claim 1, wherein the coupling agent comprising an active group is a silane compound comprising an amino group, represented by the following formula (2): 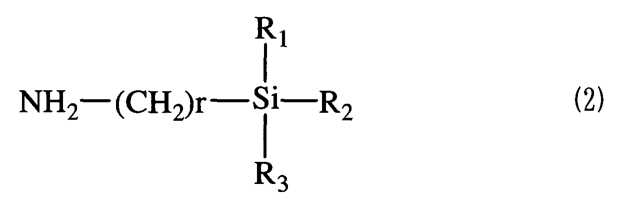 (wherein, R1, R2 and R3 represent an identical or different group being -O(CH₂)ₐCH₃ or Cl, a represents an identical or different integer from 0 to 10, and r represents an integer from 0 to 10).

3. A substrate according to claim 1, wherein the coupling agent comprising an active group, is a silane compound comprising an amino group, represented by the following formula (3): 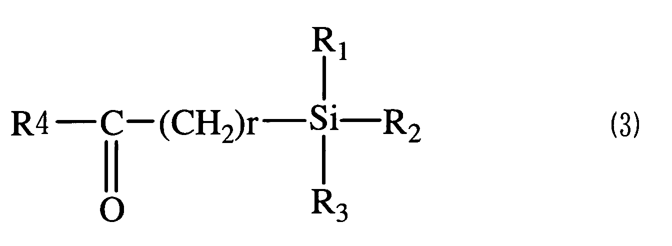 (wherein R4 represents any one of the following structures: 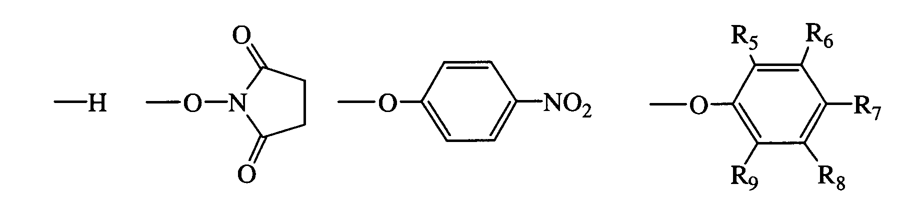 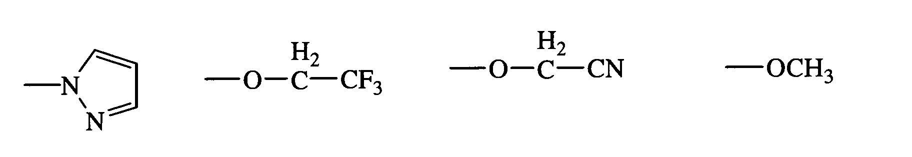 wherein, R5, R6, R7, R8 and R9 represent an identical or different atom being H, Cl or F).

4. A substrate according to claim 1, wherein the coupling agent comprising an active group is a silane compound comprising an amino group, represented by the following formula (4): 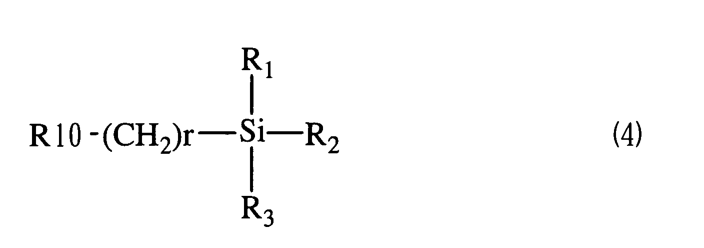 wherein R10 represents any one of the following structures:

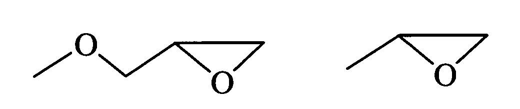

5. A substrate according to any one of claims 1 to 4, wherein the cross-linking reagent A-L-B is any one of the following: 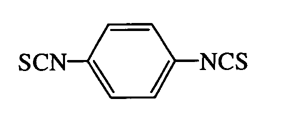 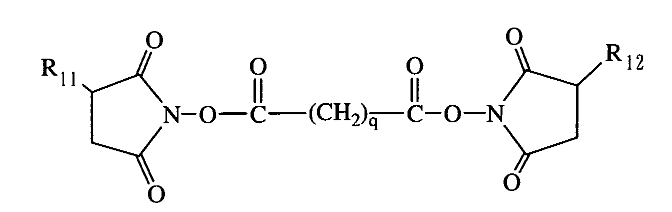 (wherein R11 and R12, represent an identical or different group being -H or -SO₃) 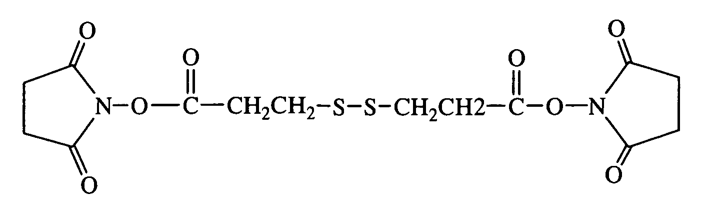 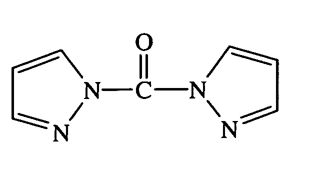 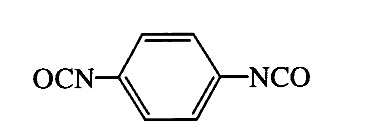
OHC(CH₂)₃CHO

6. A substrate having a branching agent intramolecularly possessing 2 or more primary amino groups, bound thereto by means of the cross-linking agent of the substrate according to any one of claims 1 to 5, represented by A-L-B of general formula (1).

7. A substrate according to claim 6, wherein the branching agent is a compound having 2 or 3 primary amino groups, which is represented by the following general formula (5): 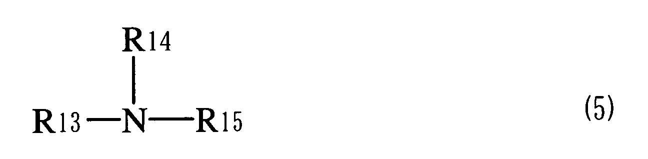 (wherein, R13, R14 and R15 represent an identical or different group selected from alkyl group having a primary amino group, alkyl group or hydrogen atom, provided that 2 or 3 are alkyl groups having a primary amino group).

8. A substrate according to claim 7, wherein R13, R14 and R15 of the general formula (5) are alkyl amino groups represented by the following general formula (6):
NH₂(CH₂)_{b}(NH)_{c}(CH₂)_{d}(NH)ₑ(CH₂)_{f}- (6)
(wherein, b, d and f represent identical or different integers from 0 to 10, c and e represent integers from 0 to 1, provided that cases where b, d and f are all 0 is not included).

9. A substrate according to claim 6, wherein the branching agent is a polyamide dendrimer having a primary amino group.

10. A substrate having a straight chain linker intramolecularly possess 2 primary amino groups bound thereto by means of the cross-linking reagent of the substrate according to any one of claims 1 to 5 represented by A-L-B of general formula (1).

11. A substrate according to claim 10, wherein the straight chain linker is a compound having 2 primary amino groups which is represented by the following general formula (7) or (8):
NH₂(CH₂)ₘO(CH₂) ₙO(CH₂) ₛO(CH₂) ₜNH₂ (7)
NH₂(CH₂)mO(CH₂) ₙO(CH₂) ₛNH₂ (8)
(wherein, m, n, s and t represent identical or different integers from 1 to 10)

12. A substrate having a cross-linking reagent of the following general formula (1) further bound thereonto by means of the branching agent intramolecularly possessing 2 or more primary amino groups according to any one of claims 6 to 11:
A-L-B (1)
(wherein, A and B represent an identical or different group which reacts with an active group of the coupling agent comprising an active group, selected from an active ester group, isothiocyanate group, isocyanate group, imidazole group, carbodiimide group or aldehyde group, and wherein L is a linking group linking A and B, selected from a straight chain alkyl group, aryl group, allyl group or alkyl group having an amide group).

13. A substrate for immobilization of a biological substance or medicament which has a biological substance or chemical substance, being an object of binding, immobilized thereon by means of the cross-linking agent of the substrate according to any one of claims 1 to 12, represented by general formula (1) A-L-B.

14. A method of immobilizing a biological substance or medicament which comprises immobilizing a biological substance or chemical substance on the substrate according to any one of claims 1 to 12.

15. A method of coating a substrate which comprises reacting a coupling agent comprising an active group on a substrate, and then reacting a cross-linking reagent represented by the following formula:
A-L-B (1)
(wherein, A and B represent an identical or different group which reacts with an active group of the coupling agent comprising an active group, selected from an active ester group, isothiocyanate group, isocyanate group, imidazole group, carbodiimide group or aldehyde group, and wherein L is a linking group linking A and B, selected from a straight chain alkyl group, aryl group, allyl group or alkyl group having an amide group).

16. A method of coating a substrate according to claim 15, wherein the coupling agent comprising an active group is a silane compound comprising an amino group, represented by the following general formula (2): 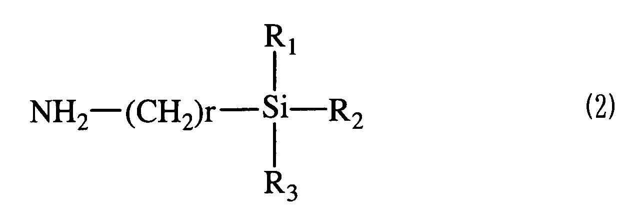 (wherein, R1, R2 and R3 represent an identical or different group being -O(CH₂)ₐCH₃ or Cl, a represents an identical or different integer from 0 to 10, and r represents an integer from 0 to 10).

17. A method of coating a substrate according to claim 15, wherein the coupling agent comprising an active group, is a silane compound comprising an amino group, represented by the following general formula (3): 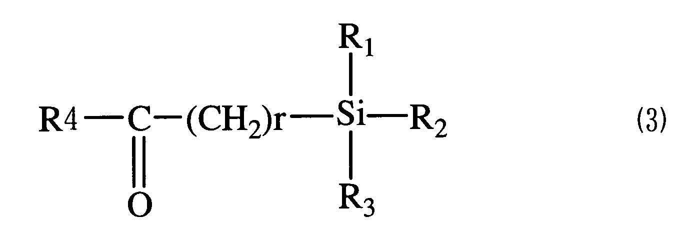 (wherein R4 represents any one of the following structures: 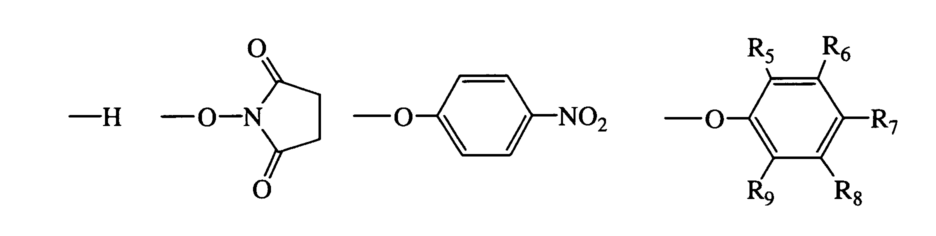 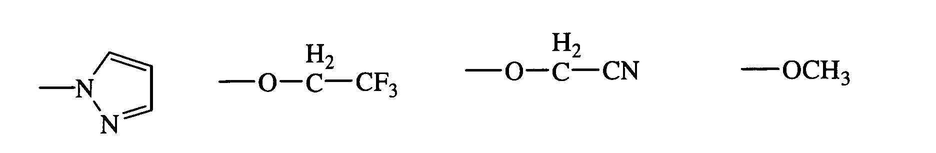 wherein, R5, R6, R7, R8 and R9 represent an identical or different atom being H, Cl or F).

18. A method of coating a substrate according to claim 15, wherein the coupling agent comprising an active group is a silane compound comprising an amino group, represented by the following formula (5): 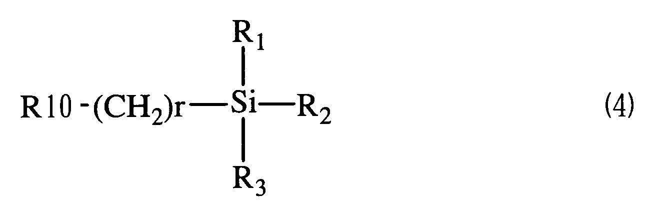 wherein R10 represents any one of the following structures:

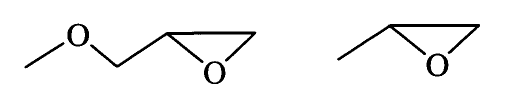

19. A method of coating a substrate according to any one of claims 15 to 18, wherein the cross-linking reagent A-L-B is any one of the following: 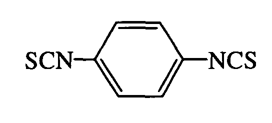 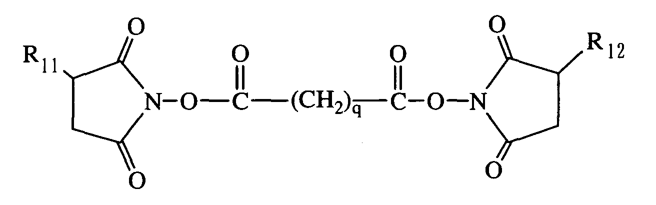 (wherein R11 and R12, are identical or different group being -H or -SO₃) 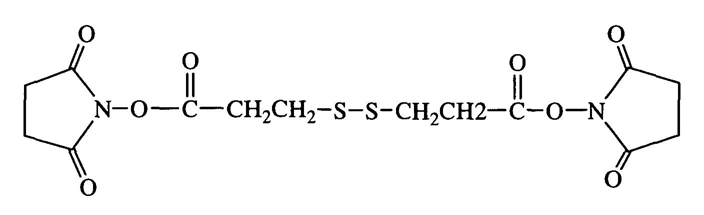 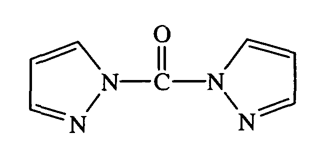 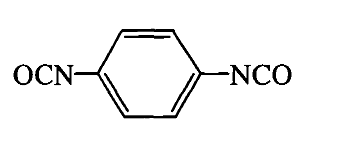
OHC(CH₂)₃CHO

20. A coating method which comprises reacting a branching agent intramolecularly possessing 2 or more primary amino groups with the substrate according to any one of claims 15 to 19.

21. A coating method according to claim 20, wherein the branching agent is a compound having 2 or 3 primary amino groups represented by the following general formula (5): 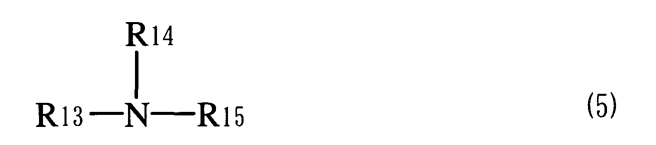 (wherein, R13, R14 and R15 are an identical or different group selected from alkyl group having a primary amino group, alkyl group or hydrogen atom, provided that 2 or 3 are alkyl groups having a primary amino group).

22. A method of coating a substrate according to claim 21, wherein R13, R14 and R15 of the general formula (5) are alkyl amino groups represented by the following general formula (6):
NH₂(CH₂)_{b}(NH)_{c}(CH₂)_{d}(NH)ₑ(CH₂)_{f}- (6)
(wherein, b, d and f represent identical or different integers from 0 to 10, c and e represent integers from 0 to 1, provided that cases where b, d and f are all 0 is not included).

23. A method of coating a substrate according to claim 20, wherein the branching agent is a polyamide dendrimer having a primary amino group.

24. A method of coating a substrate which comprises further reacting a cross-linking reagent of the following general formula (1) with the substrate according to any one of claims 21 to 23:
A-L-B (1)
(wherein, A and B represent an identical or different group which reacts with an active group of the coupling agent comprising an active group, selected from an active ester group, isothiocyanate group, isocyanate group, imidazole group, carbodiimide group or aldehyde group, and wherein L is a linking group linking A and B, selected from a straight chain alkyl group, aryl group, allyl group or alkyl group having an amide group).
